(19) Europäisches Patentamt
European Patent Office
Office européen des brevets

(11) EP 0 780 125 B1

(12) **EUROPEAN PATENT SPECIFICATION**

(45) Date of publication and mention of the grant of the patent:
**18.04.2001 Bulletin 2001/16**

(51) Int Cl.$^7$: **A61K 31/415**

(21) Application number: **96120597.8**

(22) Date of filing: **20.12.1996**

(54) **Use of hydantoin derivatives for the preparation of a medicament for the treatment of dieases associated with the presence of active oxygen and free radicals**

Verwendung von Hydantoin-Derivaten zur Herstellung eines Medikaments zur Behandlung von Krankheiten in Zusammenhang mit aktivem Sauestoff und freien Radikalen

Utilisation de dérivés d'hydantoine pour la préparation d'un médicament pour le traitement des maladies associées à la présence d'oxygène actif et de radicaux libres

(84) Designated Contracting States:
**AT BE CH DE DK ES FR GB IT LI NL SE**

(30) Priority: **20.12.1995 JP 34983195**

(43) Date of publication of application:
**25.06.1997 Bulletin 1997/26**

(73) Proprietor: **NIPPON ZOKI PHARMACEUTICAL CO., LTD.**
**Chuo-ku, Osaka (JP)**

(72) Inventors:
- **Endou, Hitoshi**
  **Sagamihara, Kanagawa (JP)**
- **Ienaga, Kazuharu,**
  **c/o Nippon Zoki Pharm. Co., Ltd.**
  **Katoh-gun, Hyogo (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 160 618**
**EP-A- 0 194 226**
**EP-A- 0 412 940**
**WO-A-86/01110**

- **PATENT ABSTRACTS OF JAPAN vol. 012, no. 444 (C-545), 22 November 1988 & JP 63 166870 A (NIPPON ZOKI PHARMACEUT CO LTD), 11 July 1988,**

EP 0 780 125 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Detailed Description of the Invention:

The present invention relates to the use of various hydantoin derivatives for the manufacture of a medicament for the treatment of diseases associated with the presence of active oxygen and free radicals.

Strong concern has been given in recent years to damage of molecules, membranes and tissues of living organisms by free radicals or by active oxygen and also to various diseases caused by that. Free radicals are chemical species having unpaired electrons and they are unstable and highly reactive. With regard to activated oxygen species, there are superoxides, hydroxyl radical, hydrogen peroxide and singlet oxygen. Among them, superoxides and hydroxyl radical are radical species and, particularly, hydroxyl radical has been known as a free radical having a very high reactivity. There are so many target molecules in living organisms damaged by free radicals and active oxygen and they are lipids, nucleic acids, enzymes, proteins and sugars. Among them, highly unsaturated fatty acids which are usually confined to lipids of cells are especially apt to be attacked resulting in an oxidative degradation whereby lipid peroxides are produced. As a result thereof, cell membranes are damaged whereupon not only the membrane structure is destroyed but also enzymatic action and acceptor function of the proteins which are present and acting there are greatly damaged. In addition, it has been known that the resulting lipid peroxides flow out from the topical region to blood causing a secondary pathology such as blood vessel pathology.

There have been many reports on the correlation of free radicals and active oxygen with the diseases and the usefulness of the substances having an ability of eliminating free radicals and active oxygen as pharmaceuticals has been greatly attracting public attention. SOD (superoxide dismutase) which is a substance eliminating the superoxides in living organisms is a representative substance which eliminates free radicals and active oxygen and its development as a pharmaceutical agent is now under way. Therapeutic targets by SOD cover a wide variety of diseases including myocardial infarction, reperfusion disturbance, autoimmune diseases (such as collagen disease, Behç}et disease and ulcerative colitis), fibrosis, pulmonary diseases (such as pulmonary edema and pulmonary fibrosis), dermatological diseases (such as burn injury, external wounds, keloid, hypersensitivity to sunlight and dermatitis), arthropathy, side effects of anticancer agents, radiation diseases, septic shock, inflammatory diseases and cataract. Refer, for example, to -Pathologic biochemistry and clinics of free radicals, inflammation and antiinflammation-, Nippon Rinsho, volume 46, number 10, pages 93-97 (1988).

SOD is a protein preparation and has some problems that its half life period in blood is short and that it is hardly incorporated into cells. Therefore, the fact at present is that various investigations on techniques for manufacturing its preparations is now being conducted. Consequently, there has been a demand for a novel agent for eliminating free radicals and active oxygen which can be easily made into pharmaceutical preparations and has little side effects.

JP-A-63-166870 discloses a use of 1-cyclohexylimidazolidinetrione for the treatment or prevention of bronchial asthma and for the treatment or prevention of other allergic disorders such as dermatosis and rhinitis.

EP-A-0 412 940 discloses the use of certain hydantoin or imidazolidinetrione derivatives for the prevention or treatment of renal failure.

WO-86/01110 discloses that absorption of oxygen free radicals by the scavenger SOD or WR-2721 is boosted by the co-administration of certain hydantoin derivatives. However, such derivatives are not taught to be pharmacologically active themselves.

An object of the present invention is to provide the use of various hydantoin derivatives for the manufacture of a medicament for treating diseases associated with the presence of free radicals and oxygen.

According to a first aspect, the present invention provides the use of a hydantoin derivative represented by formula (I) or a pharmaceutically acceptable salt thereof

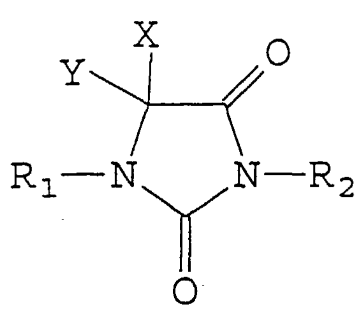

(I)

wherein each of $R_1$ and $R_2$, which may be the same or different, represents hydrogen, an alkyl group or a cycloalkyl group; and each of X and Y, which may be the same or different, represents hydrogen, hydroxyl group, an alkyl group or an alkoxy group, or X and Y together represent an oxo group, and a pharmaceutically acceptable carrier or diluent, for the preparation of a medicament for the treatment of fibrosis, dermatological diseases, arthropathy, septic shock or cataracts with the proviso that if X and Y together represent an oxo group, then the use excludes the preparation of a medicament for the treatment of dermatological diseases.

According to a second aspect, the present invention provides the use of a hydantoin derivative represented by formula (I) or a pharmaceutically acceptable salt thereof

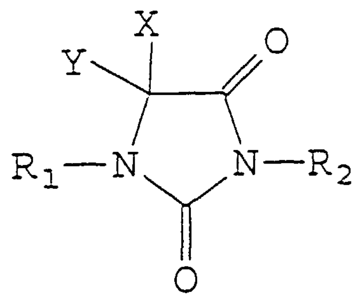

(I)

wherein each of $R_1$ and $R_2$, which may be the same or different, represents hydrogen, an alkyl group or a cycloalkyl group; and each of X and Y, which may be the same or different, represents hydrogen, hydroxyl group, an alkyl group or an alkoxy group, and a pharmaceutically acceptable carrier or diluent, for the preparation of a medicament for the treatment of autoimmune diseases, inflammatory diseases or pulmonary diseases.

According to a third aspect, the present invention provides the use of a hydantoin derivative represented by formula (I) or a pharmaceutically acceptable salt thereof

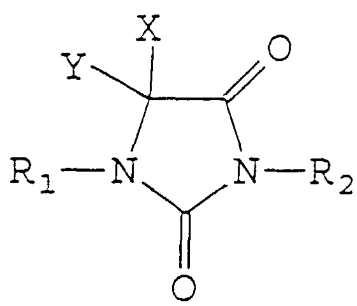

(I)

wherein each of $R_1$ and $R_2$, which may be the same or different, represents hydrogen, an alkyl group or a cycloalkyl group; and each of X and Y, which may be the same or different, represents hydrogen, hydroxyl group, an alkyl group or an alkoxy group, or X and Y together represent an oxo group, and a pharmaceutically acceptable carrier or diluent, for the preparation of a medicament containing the hydantoin derivative of formula (I) as the only active component for the treatment of myocardial infarction, reperfusion disturbance, side effects of anti-cancer agents other than renal failure, or radiation diseases.

In the above mentioned formula (I), each of $R_1$ and $R_2$, which may be the same or different, represents hydrogen, an alkyl group, preferably a straight or branched alkyl group having 1 to 20 carbon atoms such as methyl, ethyl, propyl, isopropyl, butyl, isobutyl, sec-butyl, t-butyl, pentyl, isopentyl, neopentyl, t-pentyl, hexyl, dimethylbutyl, heptyl, octyl, nonyl, decyl or stearyl; or a cycloalkyl group, preferably a cycloalkyl group having 3 to 8 carbon atoms such as cyclopropyl, cyclobutyl, cyclopentyl, cyclohexyl, cycloheptyl or cyclooctyl. Each of X and Y, which may be the same or different, represents hydrogen, hydroxyl group, an alkyl group, preferably a straight or branched alkyl group having 1 to 3 carbon atoms such as methyl, ethyl, propyl, isopropyl; or an alkoxy group, preferably a straight or branched alkoxy group having 1 to 5 carbon atoms such as methoxy, ethoxy, propoxy, isopropoxy, butoxy, isobutoxy, sec-butoxy, t-butoxy, pentoxy, isopentoxy, neopentoxy; or, in the first and third aspects, X and Y together may represent an oxo group.

Preferred compounds for use in the present invention are indicated as follows.

[Compound 1] Hydantoin

[Compound 2] 1-Methylhydantoin
[Compound 3] 3-Methylhydantoin
[Compound 4] 1-Ethylhydantoin
[Compound 5] 1-Propylhydantoin
[Compound 6] 1-Butylhydantoin
[Compound 7] 1-t-Butylhydantoin
[Compound 8] 1-Hexylhydantoin
[Compound 9] 1-(1,3-Dimethylbutyl) hydantoin
[Compound 10] 1-Decylhydantoin
[Compound 11] 1-Stearylhydantoin
[Compound 12] 1,3-Dimethylhydantoin
[Compound 13] 1,5-Dimethylhydantoin
[Compound 14] 3,5-Dimethylhydantoin
[Compound 15] 1-Cyclopentylhydantoin
[Compound 16] 1-Cyclohexylhydantoin
[Compound 17] l-Cyclohexyl-3-methylhydantoin
[Compound 18] 3-Cyclohexylhydantoin
[Compound 19] 1,3-Dicyclohexylhydantoin
[Compound 20] 5-Hydroxyhydantoin
[Compound 21] 5-Hydroxy-l-methylhydantoin
[Compound 22] 5-Hydroxy-3-methylhydantoin
[Compound 23] 5-Hydroxy-l-ethylhydantoin
[Compound 24] 5-Hydroxy-l-propylhydantoin
[Compound 25] 5-Hydroxy-1-butylhydantoin
[Compound 26] 5-Hydroxy-l-t-butylhydantoin
[Compound 27] 5-Hydroxy-1-hexylhydantoin
[Compound 28] 5-Hydroxy-1-(1,3-dimethylbutyl)hydantoin
[Compound 29] 5-Hydroxy-1-decylhydantoin
[Compound 30] 5-Hydroxy-1-stearylhydantoin
[Compound 31] 5-Hydroxy-1-cyclopentylhydantoin
[Compound 32] 5-Hydroxy-1-cyclohexylhydantoin
[Compound 33] 5-Hydroxy-1-cyclohexyl-3-methylhydantoin
[Compound 34] 5-Hydroxy-1,3-dimethylhydantoin
[Compound 35] 5-Hydroxy-1,5-dimethylhydantoin
[Compound 36] 5-Hydroxy-3,5-dimethylhydantoin
[Compound 37] 5-Hydroxy-1,3-dicyclohexylhydantoin
[Compound 38] 5-Methoxyhydantoin
[Compound 39] 5-Methoxy-1-methylhydantoin
[Compound 40] 5-Methoxy-3-methylhydantoin
[Compound 41] 5-Methoxy-1-ethylhydantoin
[Compound 42] 5-Methoxy-1-propylhydantoin
[Compound 43] 5-Methoxy-1-butylhydantoin
[Compound 44] 5-Methoxy-1-cyclohexylhydantoin
[Compound 45] 5-Methoxy-3-cyclohexylhydantoin
[Compound 46] 5-Ethoxyhydantoin
[Compound 47] 5-Ethoxy-1-methylhydantoin
[Compound 48] 5-Ethoxy-3-methylhydantoin
[Compound 49] 5-Ethoxy-1-ethylhydantoin
[Compound 50] 5-Ethoxy-1-propylhydantoin
[Compound 51] 5-Ethoxy-1-butylhydantoin
[Compound 52] 5-Propoxyhydantoin
[Compound 53] 5-Propoxy-1-methylhydantoin
[Compound 54] 5-Propoxy-3-methylhydantoin
[Compound 55] 5-Propoxy-1-ethylhydantoin
[Compound 56] 5-Propoxy-1-propylhydantoin
[Compound 57] 5-Propoxy-1-butylhydantoin
[Compound 58] 5-Butoxyhydantoin
[Compound 59] 5-Butoxy-1-methylhydantoin

[Compound 60] 5-Butoxy-3-methylhydantoin
[Compound 61] 5-t-Butoxyhydantoin
[Compound 62] 5-t-Butoxy-1-methylhydantoin
[Compound 63] 5-t-Butoxy-3-butylhydantoin
[Compound 64] Imidazolidinetrione
[Compound 65] 1-Methylimidazolidinetrione
[Compound 66] 1-Ethylimidazolidinetrione
[Compound 67] 1-Butylimidazolidinetrione
[Compound 68] 1-Isobutylimidazolidinetrione
[Compound 69] 1-t-Butylimidazolidinetrione
[Compound 70] 1-Hexylimidazolidinetrione
[Compound 71] 1-(1,3-Dimethylbutyl)imidazolidinetrione
[Compound 72] 1-Decylimidazolidinetrione
[Compound 73] 1-Cyclopentylimidazolidinetrione
[Compound 74] 1-Cyclopentyl-3-ethylimidazolidinetrione
[Compound 75] 1-Cyclohexylimidazolidinetrione
[Compound 76] 1,3-Dimethylimidazolidinetrione
[Compound 77] 1,3-Dicyclohexylimidazolidinetrione

The hydantoin derivatives of the present invention include the pharmaceutically acceptable salts of the compounds represented by the above given formula (I) such as acid addition salts with hydrochloric acid, sulfuric acid, nitric acid, hydrobromic acid, phosphoric acid, perchloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid, sulfanilic acid, or salts with alkali metal such as sodium and potassium, salts with alkaline-earth metal such as calcium, magnesium and barium, salts with other metals such as aluminum and zinc. Those salts may be manufactured by known methods from the hydantoin derivatives of the present invention in a free state or may be mutually converted among the salts.

When there are steric isomers such as cis-trans isomer, optical isomer, conformational isomer, hydrate and complex for the substances of the present invention, the present invention includes any and all of them. The compounds of the present invention and the methods for manufacturing them are disclosed, for example, in Japanese Laid Open (Kokai) Nos. 61/122275 and 62/14.

The substance of the present invention can be made into pharmaceutical preparations by a combination with a suitable pharmaceutical carriers or diluents. Any of the known methods for providing preparations, such as for oral administrations (e.g. tablets, capsules, powders or liquids) and for parenteral administrations (e.g. for subcutaneous, intravenous, intramuscular, intrarectal and intranasal administrations) may be used to produce the pharmaceutical compositions of the present invention. In preparing the preparations, the substance of the present invention may be used in the form of their pharmaceutically acceptable salts, and also can be used either solely or jointly together with other pharmaceutically active components.

In the case of preparations for oral administration, the substance of the present invention as it is or together with commonly-used excipients such as a suitable additive (e.g. lactose, mannitol, corn starch, potato starch or potassium citrate) is mixed with binders such as cellulose derivatives (e.g. crystalline cellulose or hydroxypropylcellulose), gum arabicum, corn starch or gelatin, disintegrating agents such as corn starch, potato starch or calcium carboxymethylcellulose, lubricating agents such as talc or magnesium stearate and others including bulking agents, moisturizing agents, buffers, preservatives or perfumes to give tablets, diluted powders, granules or capsules.

In the case of injections, it is possible to prepare the solutions or the suspensions in an aqueous and nonaqueous solvents such as distilled water for injection, physiological saline solution, Ringer's solution, plant oil, synthetic fatty acid glycerides, higher fatty acid esters or propylene glycol.

It is also possible, depending upon the type of the disease, to prepare the pharmaceutical preparations which are other than those which were mentioned already and are suitable for the therapy such as, for example, syrup, suppositories, inhalations, aerosol preparations, collyriums or medicines for external use (e.g. ointments, gels, poultices).

The preferred dose of the substance of the present invention may vary depending upon the object to be administered the patient, form of the preparation, method for the administration or term for the administration and, in order to achieve a desired effect, 1-1,000 mg per day, preferably 5-600 mg per day may be usually given to common adults by oral route. In the case of a parenteral administration such as by injection, it is preferred that, due to the influence, e.g. of the absorption, a level of from 1/3 to 1/10 of the above given dose by oral route is administered.

An example of a pharmaceutical formulation containing the compounds of the present invention as an effective

component is given below.

TABLE 1.

| Tablet formulation | |
|---|---|
| **Components** | **Amount per tablet** |
| Compound of the present<br>invention<br>Lactose<br>Crystalline cellulose<br>Hydroxypropylcellulose Magnesium stearate | 100 mg<br>35 mg<br>5 mg<br>5 mg<br>5 mg |
| **Total** | **150 mg** |

Preferred embodiments of the eliminating agent for active oxygen and free radicals containing the compound of the present invention represented by the above given formula (I) are given as follows.

(1) An eliminating agent for active oxygen and free radicals containing the compound represented by the formula (I) wherein one of X and Y represents hydroxyl group.
(2) An agent according above given (1) wherein the other of X and Y represents hydrogen.
(3) An agent according above given (2) wherein one of $R_1$ and $R_2$ represents an alkyl group and the other represents hydrogen.
(4) An agent according above given (3) wherein $R_1$ represents an alkyl group.
(5) An agent according above given (4) wherein $R_1$ represents an alkyl group having 1 to 4 carbon atoms.
(6) An agent according above given (5) wherein $R_1$ represents methyl.
(7) An eliminating agent for active oxygen and free radicals containing the compound represented by the formula (I) wherein both of X and Y represent hydrogens.
(8) An agent according above given (7) wherein one of $R_1$ and $R_2$ represents an alkyl group and the other represents hydrogen.
(9) An agent according above given (8) wherein $R_1$ represents an alkyl group.
(10) An agent according above given (9) wherein $R_1$ represents an alkyl group having 1 to 4 carbon atoms.
(11) An agent according above given (10) wherein $R_1$ represents methyl.
(12) An agent according above given (1) wherein the other of X and Y represents an alkoxy group.
(13) An agent according above given (12) wherein $R_1$ represents an alkyl group and $R_2$ represents hydrogen.
(14) An agent according above given (13) wherein $R_1$ represents methyl.
(15) An agent according above given (1) wherein the other of X and Y represents an alkyl group.
(16) An agent according above given (15) wherein $R_1$ represents an alkyl group and $R_2$ represents hydrogen.
(17) An agent according above given (16) wherein $R_1$ represents methyl.
(18) An eliminating agent for active oxygen and free radicals containing the compound represented by the formula (I) wherein X and Y together represent an oxo group (except for the treatment of dermatological diseases).
(19) An agent according above given (18) wherein $R_1$ represents an alkyl group and $R_2$ represents hydrogen.
(20) An agent according above given (19) wherein $R_1$ represents methyl.
(21) An eliminating agent for hydroxyl radicals containing the compound represented by the formula (I).
(22) An eliminating agent for hydroxyl radicals containing the compound defined in any of above given (1) to (20).

[Examples]

(1) Measurement of ability for eliminating hydroxyl radicals.

Ability of the test substances for eliminating hydroxyl radicals was measured using an ESR spin trapping method which is frequently used for the detection of hydroxyl radicals. Generation of hydroxyl radicals was carried out using a Fenton's reaction in which hydroxyl radicals are generated by a reaction of divalent iron chelated with DTPA (diethylenediamine-pentaacetic acid) with hydrogen peroxide. DMPO (dimethylpyrroline N-oxide) promptly reacts with the hydroxyl radicals generated thereby and the resulting reaction product shows an ESR signal having four lines with a characteristic intensity ratio of 1:2:2:1. When a test substance having an eliminating action for hydroxyl radicals is made coexisting in this reaction system, the above characteristic signal is inhibited and, therefore, the ability for eliminating hydroxyl radicals can be measured using that as an index.

Thus, a solution of a mixture of 1 mM of ferrous sulfate and DTPA dissolved in 100 mM of a phosphate buffer (pH 7.8) was charged in a test tube, then 50 ml of a sample solution containing the test substance and 20 ml of 1 mM or 2 mM of DMPO were added and, finally, 75 ml of 1 mM of hydrogen peroxide solution was added thereto whereby the reaction was initiated. Hydrogen peroxide solution was prepared immediately before each of the experiments and the reaction was conducted at room temperature. An ESR spectrum in this reaction system was measured by conventional means. Concentration ($IC_{50}$) of the test substance when the signal intensity by DMPO-OH was inhibited to an extent of 50 % in the presence of the test substance as compared with that in the absence of that was determined.

An example of the results when 1 mM of DMPO was used will be given as hereunder. Dimethyl sulfoxide which has been known to have an eliminating action for hydroxyl radicals was used as a control for comparison.

Table 2

| Test substance | $IC_{50}$ (M) |
|---|---|
| Dimethyl sulfoxide | $1.6 \times 10^{-4}$ |
| Compound 21 | $1.0 \times 10^{-3}$ |

(2) Inhibitory action against damage of endothelial cells caused by activated leucocytes.

Inhibitory action of the compounds of the present invention against damage of cells caused by hydroxyl radicals was measured using the following experimental system.

Endothelial cells of blood vessel obtained from bovine descending aorta by an enzymatic treatment was cultured in a 48 well plate using an MEM Eagle containing 15 % of fetal calf serum. Then 3.7 KBq of $^{51}Cr$ (sodium chromate) was added to the endothelial cells in the confluent state and culturing was conducted for 18 hours whereby $^{51}Cr$ was incorporated into the endothelial cells. Chromium in the medium was washed out and the test substance, human leucocytes and phorbol ester (phorbol myristate acetate) were added thereto followed by culturing for six hours. Radiation activity of chromium liberated into the medium from the endothelial cells damaged by the activated leucocytes was measured by a gamma-ray counter and the degree of damage of the endothelial cells was calculated by the following formula.

$$\text{(Degree of damage of endothelial cells} = \frac{\text{(Sample treated with the activated leucocytes in the presence of the test substance) - (Sample without any treatment)}}{\text{(Sample treated with Triton X-100) - (Sample without any treatment)}}$$

The degree of damage of endothelial cells for the activated leucocytes only, i.e. in the absence of the test substance, was settled to 100% as a control, and an example of the results is given in Fig. 1.

From the above mentioned test results, it has now been clear that the substance of the present invention has an action of eliminating hydroxyl radicals. When concentration of the adding DMPO is changed in said pharmacological test (1), the $IC_{50}$ of the compound of the present invention changes accordingly and, therefore, it has been suggested that the eliminating action of the compound of the present invention for hydroxyl radicals is a competitive antagonism to DMPO.

Consequently, the hydantoin derivatives of the present invention having an eliminating action to active oxygen and free radicals are useful as pharmaceuticals for treating a wide variety of diseases in which active oxygen or free radicals are participated including myocardial infarction, reperfusion disturbance, autoimmune diseases (such as collagen disease, Behç}et disease and ulcerative colitis), fibrosis, pulmonary diseases (such as pulmonary edema and pulmonary fibrosis), dermatological diseases (such as burn injury, external wounds, keloid, hypersensitivity to sunlight and dermatitis), arthropathy, side effects of anticancer agents, radiation diseases, septic shock, inflammatory diseases and cataract which have been known as the diseases to be treated with SOD. Unlike dimethyl sulfoxide and the like having cytotoxic action, the compounds of the present invention have very low toxicity and high safety and are capable of being administered by oral route. Therefore, they can be used to chronic diseases which need administration for long period as well and their usefulness as pharmaceuticals is high.

Brief Explanation of the Figure:

Fig. 1 shows an example of the results of inhibitory action of the compound of the present invention against damage of cells caused by hydroxyl radicals.

## Claims

1. Use of a hydantoin derivative represented by formula (I) or a pharmaceutically acceptable salt thereof

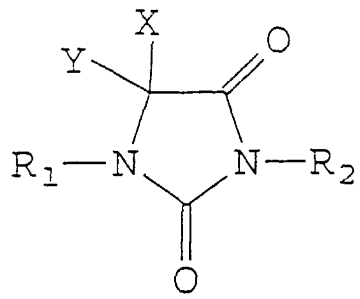

(I)

wherein each of $R_1$ and $R_2$, which may be the same or different, represents hydrogen,-an alkyl group or a cycloalkyl group; and each of X and Y, which may be the same or different, represents hydrogen, hydroxyl group, an alkyl group or an alkoxy group, or X and Y together represent an oxo group, and a pharmaceutically acceptable carrier or diluent, for the preparation of a medicament for the treatment of fibrosis, dermatological diseases, arthropathy, septic shock or cataracts with the proviso that if X and Y together represent an oxo group, then the use excludes the preparation of a medicament for the treatment of dermatological diseases.

2. Use of a hydantoin derivative represented by formula (I) or a pharmaceutically acceptable salt thereof

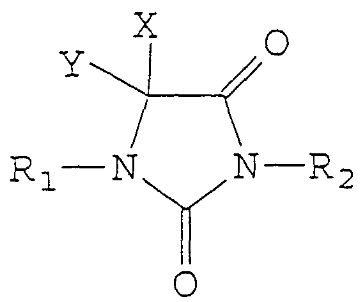

(I)

wherein each of $R_1$ and $R_2$, which may be the same or different, represents hydrogen, an alkyl group or a cycloalkyl group; and each of X and Y, which may be the same or different, represents hydrogen, hydroxyl group, an alkyl group or an alkoxy group, and a pharmaceutically acceptable carrier or diluent, for the preparation of a medicament for the treatment of autoimmune diseases, inflammatory diseases or pulmonary diseases.

3. Use of a hydantoin derivative represented by formula (I) or a pharmaceutically acceptable salt thereof

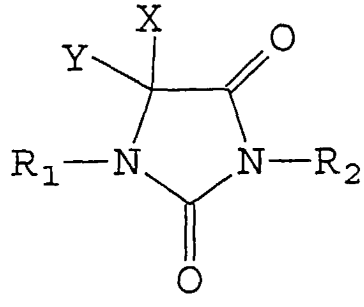

(I)

wherein each of $R_1$ and $R_2$, which may be the same or different, represents hydrogen, an alkyl group or a cycloalkyl group; and each of X and Y, which may be the same or different, represents hydrogen, hydroxyl group, an alkyl group or an alkoxy group, or X and Y together represent an oxo group, and a pharmaceutically, acceptable carrier or diluent, for the preparation of a medicament containing the hydantoin derivative of formula (I) as the only active component for the treatment of myocardial infarction, reperfusion disturbance, side effects of anti-cancer agents other than renal failure, or radiation diseases.

**4.** Use according to any of Claims 1 to 3, wherein the pharmaceutical composition is to be administered orally.

**5.** Use according to any of Claims 1 to 4, wherein one of X and Y is a hydroxyl group.

**6.** Use according to Claim 5, wherein the other of X and Y is hydrogen.

**7.** Use according to Claim 6, wherein one of $R_1$ and $R_2$ is an alkyl group and the other is hydrogen.

**8.** Use according to Claim 7, wherein $R_1$ is methyl.

**Patentansprüche**

**1.** Verwendung eines Hydantoinderivats, dargestellt durch Formel (I) oder eines pharmazeutisch annehmbaren Salzes davon:

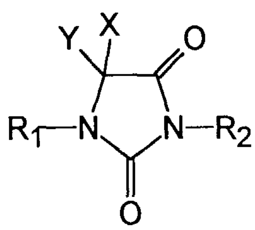

(I)

worin jedes der $R_1$ und $R_2$, die gleich oder verschieden sein können, Wasserstoff, eine Alkylgruppe oder eine Cycloalkylgruppe darstellt; und jedes der X und Y, die gleich oder verschieden sein können, Wasserstoff, eine Hydroxygruppe, eine Alkylgruppe oder eine Alkoxygruppe darstellt, oder X und Y stelen zusammen eine Oxogruppe dar, und einen pharmazeutisch annehmbaren Träger oder Diluens für die Herstellung eines Medikaments für die Behandlung von Fibrose, dermatologischen Krankheiten, Arthopathie, septischem Schock oder Katarakt; mit dem Proviso, dass, wenn X und Y zusammen eine Oxogruppe darstellen, dann die Verwendung die Herstellung eines Medikaments für die Behandlung von dermatologischen Krankheiten ausschliesst.

**2.** Verwendung eines Hydantoinderivats, dargestellt durch Formel (I) oder eines pharmazeutisch annehmbaren Salzes davon

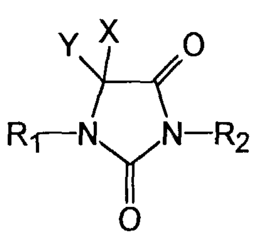

(I)

worin jedes der $R_1$ und $R_2$, die gleich oder verschieden sein können, Wasserstoff, eine Alkylgruppe oder eine Cycloalkylgruppe darstellt, und jedes der X und Y, die gleich oder verschieden sein können, Wasserstoff, eine Hydroxygruppe, eine Alkylgruppe oder eine Alkoxygruppe darstellt, und einen pharmazeutisch annehmbaren Träger oder Diluens, für die Herstellung eines Medikaments zur Behandlung von Autoimmunkrankheiten, Entzündungskrankheiten oder Lungenkrankheiten.

3. Verwendung eines Hydantoinderivats, dargestellt durch Formel (I) oder eines pharmazeutisch annehmbaren Salzes davon

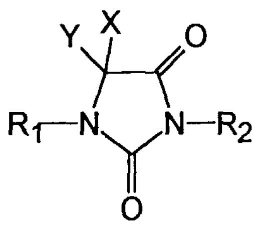

(I)

worin jedes der $R_1$ und $R_2$, die gleich oder verschieden sein können, Wasserstoff, eine Alkylgruppe oder eine Cycloalkylgruppe darstellt; und jedes der X und Y, die gleich oder verschieden sein können, Wasserstoff, eine Hydroxygruppe, eine Alkylgruppe oder eine Alkoxygruppe darstellt, oder X und Y zusammen eine Oxogruppe darstellen, und einen pharmazeutisch annehmbaren Träger oder Diluens für die Herstellung eines Medikaments, enthaltend das Hydantoinderivat der Formel (I) als die einzig aktive Komponente zur Behandlung von Herzinfarkt, Reperfusionsstörung, Nebeneffekten von Antikrebsmitteln, ausser Nierenversagen oder Strahlungsschäden.

4. Verwendung gemäss irgendeinem der Ansprüche 1 bis 3, worin die pharmazeutische Zusammensetzung oral verabreicht wird.

5. Verwendung gemäss irgendeinem der Ansprüche 1 bis 4, worin eines der X und Y eine Hydroxygruppe bedeutet.

6. Verwendung gemäss Anspruch 5, worin das andere der X und Y Wasserstoff bedeutet.

7. Verwendung gemäss Anspruch 6, worin eines von $R_1$ und $R_2$ eine Alkylgruppe und das andere Wasserstoff bedeutet.

8. Verwendung gemäss Anspruch 7, worin $R_1$ Methyl bedeutet.

## Revendications

1. Utilisation d'un dérivé de l'hydantoïne représenté par la formule (I), ou d'un de ses sels pharmaceutiquement acceptables,

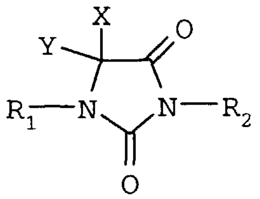

(I)

formule dans laquelle chacun des $R_1$ et $R_2$, qui peuvent être identiques ou différents, représente un hydrogène, un groupe alkyle ou un groupe cycloalkyle ; et chacun des X et Y, qui peuvent être identiques ou différents, représente un hydrogène, un groupe hydroxyle, un groupe alkyle ou un groupe alcoxy, ou X et Y représentent ensemble un groupe oxo, et d'un support ou d'un diluant pharmaceutiquement acceptable pour la préparation d'un médicament pour le traitement d'une fibrose, de maladies dermatologiques, d'une arthropathie, d'un choc septique ou d'une cataracte ; sous réserve que, si X et Y représentent ensemble un groupe oxo, l'utilisation exclut alors la préparation d'un médicament pour le traitement de maladies dermatologiques.

2. Utilisation d'un dérivé de l'hydantoïne représenté par la formule (I), ou d'un de ses sels pharmaceutiquement acceptables,

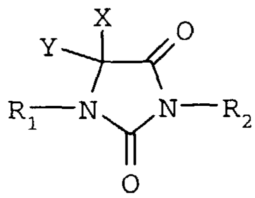

(I)

formule dans laquelle chacun des $R_1$ et $R_2$, qui peuvent être identiques ou différents, représente un hydrogène, un groupe alkyle ou un groupe cycloalkyle ; et chacun des X et Y, qui peuvent être identiques ou différents, représente un hydrogène, un groupe hydroxyle, un groupe alkyle ou un groupe alcoxy, et d'un support ou d'un diluant pharmaceutiquement acceptable pour la préparation d'un médicament pour le traitement de maladies autoimmunes, de maladies inflammatoires ou de maladies pulmonaires.

**3.** Utilisation d'un dérivé de l'hydantoïne représenté par la formule (I), ou d'un de ses sels pharmaceutiquement acceptables,

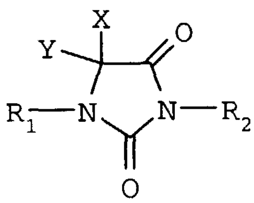

(I)

formule dans laquelle chacun des $R_1$ et $R_2$, qui peuvent être identiques ou différents, représente un hydrogène, un groupe alkyle ou un groupe cycloalkyle ; et chacun des X et Y, qui peuvent être identiques ou différents, représente un hydrogène, un groupe hydroxyle, un groupe alkyle ou un groupe alcoxy, ou X et Y représentent ensemble un groupe oxo, et d'un support ou d'un diluant pharmaceutiquement acceptable pour la préparation d'un médicament contenant le dérivé de l'hydantoïne de formule (I) en tant qu'unique composant actif pour le traitement de l'infarctus du myocarde, d'un trouble de reperfusion, d'effets secondaires d'agents anticancéreux autres qu'une insuffisance rénale ou de maladies induites par les rayonnements.

**4.** Utilisation selon une quelconque des revendications 1 à 3, dans laquelle la composition pharmaceutique est destinée à une administration par voie orale.

**5.** Utilisation selon une quelconque des revendications 1 à 4, dans laquelle un des X et Y est un groupe hydroxyle.

**6.** Utilisation selon la revendication 5, dans laquelle l'autre des X et Y est un hydrogène.

**7.** Utilisation selon la revendication 6, dans laquelle un des $R_1$ et $R_2$ est un groupe alkyle et l'autre est un hydrogène.

**8.** Utilisation selon la revendication 7, dans laquelle $R_1$ est un méthyle.

Fig. 1

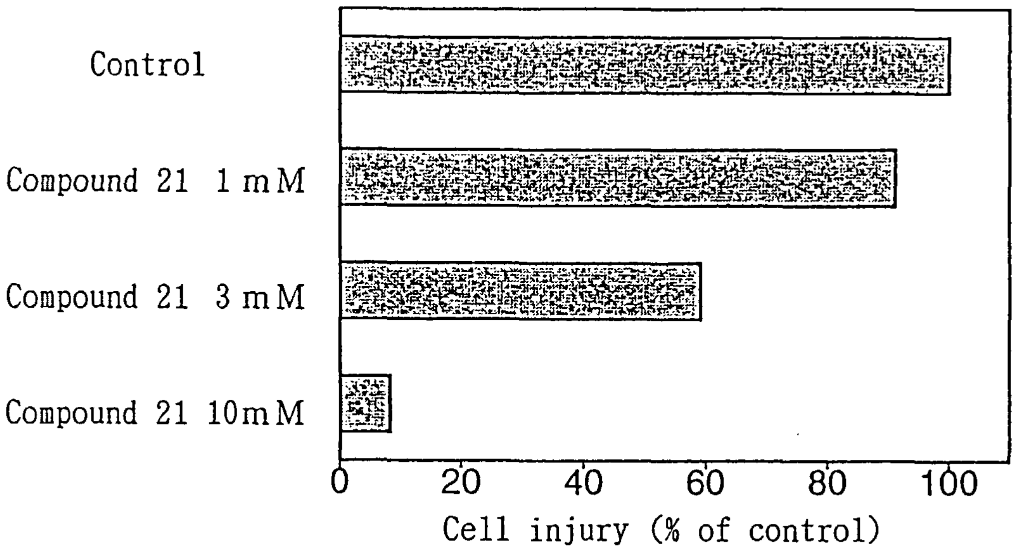
Control
Compound 21 1 mM
Compound 21 3 mM
Compound 21 10mM
0
20
40
60
80
100
Cell injury (% of control)